# EUROPEAN PATENT SPECIFICATION

(11) **EP 3 595 634 B1**
(45) Date of publication and mention of the grant of the patent: **27.03.2024**
(21) Application number: 18768248.9
(22) Date of filing: 16.03.2018
(51) Int. Cl.: A61K 9/00, A61K 48/00, C12N 9/64, C12N 15/63, A01K 67/027, A61P 27/16, C12N 15/86, C07K 14/47

(54) **GENE THERAPY CONSTRUCTS AND METHODS FOR TREATMENT OF HEARING LOSS**
GENTHERAPIEKONSTRUKTE UND VERFAHREN ZUR BEHANDLUNG VON HÖRVERLUST
CONSTRUCTIONS POUR THÉRAPIE GÉNIQUE ET PROCÉDÉS DE TRAITEMENT DE LA PERTE AUDITIVE

(30) Priority: 17.03.2017 US 201762472790 P; 12.07.2017 US 201762531522 P
(43) Date of publication of application: 22.01.2020
(73) Proprietor: Rescue Hearing Inc, Gainsville, FL 32605 (US)
(72) Inventor: STAECKER, Hinrich, Leawood KS 66209 (US); LIU, Xue, Zhong, Miami FL 33156 (US); CHEN, Zheng-Yi, Somerville MA 02143 (US); AYALA, Caesar, James, Gainesville FL 32605 (US)
(74) Representative: Manitz Finsterwald Patent- und Rechtsanwaltspartnerschaft mbB
(86) International application number: PCT/US2018/022873
(87) International publication number: WO 2018/170402

(56) References cited:
- WO-A1-2004/099779
- WO-A1-2011/075838
- WO-A1-2017/100791
- WO-A2-2007/021423
- US-A1- 2013 095 071
- US-A1- 2015 050 354
- US-B1- 7 071 004
- NICOLAS GRILLET ET AL: "Mutations in LOXHD1, an Evolutionarily Conserved Stereociliary Protein, Disrupt Hair Cell Function in Mice and Cause Progressive Hearing Loss in Humans", AMERICAN JOURNAL OF HUMAN GENETICS, vol. 85, no. 3, September 2009 (2009-09), pages 328-337, XP055732361, US ISSN: 0002-9297, DOI: 10.1016/j.ajhg.2009.07.017
- ULRICH M?LLER ET AL: "New treatment options for hearing loss", NATURE REVIEWS DRUG DISCOVERY, vol. 14, no. 5, 20 March 2015 (2015-03-20) , pages 346-365, XP055296071, GB ISSN: 1474-1776, DOI: 10.1038/nrd4533
- OHLEMILLER KEVIN K ET AL: "Application of Mouse Models to Research in Hearing and Balance", JOURNAL OF THE ASSOCIATION FOR RESEARCH IN OTOLARYNGOLOGY, SPRINGER, NEW YORK, NY, US, vol. 17, no. 6, 17 October 2016 (2016-10-17), pages 493-523, XP036101750, ISSN: 1525-3961, DOI: 10.1007/S10162-016-0589-1 [retrieved on 2016-10-17]
- SCOTT et al.: "Insertion of beta-satellite repeats identifies a transmembrane protease causing both congenital and childhood onset autosomal recessive deafness", Nature Genetics, vol. 27, no. 1, 31 January 2001 (2001-01-31), pages 59-63, XP002292771,

## Description

### CROSS-REFERENCE TO RELATED APPLICATIONS

This application claims the benefit to U.S. Provisional Application No. 62/472,790, filed March 17, 2017, and U.S. Provisional Application No. 62/531,522, filed July 12, 2017.

### FIELD OF THE DISCLOSURE

Disclosed are pharmaceutical compositions and methods useful in the treatment and/or prevention of hearing loss and deafness caused by genetic mutation of the TMPRSS3 gene or the LOXHD1 gene.

### BACKGROUND OF THE DISCLOSURE

Hearing loss is the most common sensory deficit in humans. According to 2012 estimates on the magnitude of disabling hearing loss released by the World Health Organization (WHO), there are 360 million persons worldwide living with disabling hearing loss (328 million adults and 32 million children) and 89% (or 322 million) of those persons reside in low income countries (WHO global estimates on prevalence of hearing loss; *Mortality and Burden of Diseases and Prevention of Blindness and Deafness,* WHO 2012).

There are currently no approved therapeutic agents for preventing or treating hearing loss or deafness. The current treatment option for those with disabling hearing loss is a cochlear implant. Cochlear implantation is a common procedure with a large associated healthcare cost, over $1,000,000 lifetime cost per patient (Mohr PE, et al. (2000). The societal costs of severe to profound hearing loss in the United States; Int J Technol Assess Health Care; 16(4):1120-35).

The current demand for cochlear implants exceeds supply. The production rate of cochlear implant units manufactured is 50,000 units each year. Based on current birth rates and the incidence and prevalence of disabling hearing loss in newborns, 134,000 cochlear implants are needed annually to provide 1 cochlear implant for each afflicted child. This number increases if patients needing bilateral (2) cochlear implants are included.

The lifetime cost of a cochlear implant is prohibitive for most people and particularly for those living outside the developed nations where the majority of persons with disabling hearing loss reside. Therapeutic options are needed to provide cost effective alternatives to cochlear implants, especially for those persons living outside developed nations.

More than 50% of prelingual deafness is genetic i.e. hereditary (Centers for Disease Control and Prevention- *Genetics of Hearing Loss*)*.* Hereditary hearing loss and deafness may be conductive, sensorineural, or a combination of both; syndromic (associated with malformations of the external ear or other organs or with medical problems involving other organ systems) or nonsyndromic (no associated visible abnormalities of the external ear or any related medical problems); and prelingual (before language develops) or postlingual (after language develops) (Richard JH Smith, MD, A Eliot Shearer, Michael S Hildebrand, PhD, and Guy Van Camp, PhD, Deafness and Hereditary Hearing Loss Overview, GeneReviews Initial Posting: February 14, 1999; Last Revision: January 9, 2014. More than 70% of hereditary hearing loss is nonsyndromic. The different gene loci for nonsyndromic deafness are designated DFN (for DeaFNess). Loci are named based on mode of inheritance: DFNA (Autosomal dominant), DFNB (Autosomal recessive) and DFNX (X-linked). The number following the above designations reflects the order of gene mapping and/or discovery. In the general population, the prevalence of hearing loss increases with age. This change reflects the impact of genetics and environment and the interactions between environmental triggers and an individual's genetic predisposition.

Sensorineural hearing loss (SNHL) is the most common neurodegenerative disease in humans and there are currently no approved pharmacologic interventions. SNHL can be caused by genetic disorders as well as acquired through injuries such as sound trauma and ototoxicity. Genetic diagnostics have demonstrated that there are at least 100 genes causing nonsyndromic SNHL. Recent advances in genetics and gene therapy techniques have shown that rescue of a number of recessive types of deafness is possible through gene therapy (Akil et al., 2012; Askew et al., 2015). Long term gene delivery to the inner ear has been achieved using adeno associated viral vectors (AAV) (Shu, Tao, Wang, et al., 2016). Despite the first of these observations being described several years ago, translation into a human clinical trial has not yet occurred. An ideal disease target for translational research in this domain is a recessive genetic hearing loss that affects a defined group of cells within the inner ear and occurs postnatally after the development of speech. Prevalence of the mutation is an additional consideration.

As described herein, by carefully evaluating both the incidence of common recessive causes of hearing loss and taking into account the size of the gene, it is possible to develop a gene therapy program that has an accessible and fairly common patient population. For example, although less common than other mutations, TMPRSS3 is a fairly common cause of hearing loss that is severe enough to warrant cochlear implantation. Additionally, patients with mutations in TMPRSS3 may not respond to cochlear implantation as well as patients with other mutations (Shearer et al., 2017). This presents the opportunity of targeting TMPRSS3, or other genes such as LOXHD1, as a stand-alone therapeutic or in combination with other therapeutic agents and/or cochlear implantation to improve implant outcomes for this disorder. Table 1 (adapted from (Miyagawa, Nishio, & Usami, 2016)) demonstrates that mutations in TMPRSS3 may be the most common cause of postlingual recessive hearing loss that has a fairly limited distribution within the cochlea and, due to the size of the gene, may be built into existing AAV vectors.

**Table 1: Incidence of different mutations in 176 adult cochlear implant patients**

| | ONSET | | 113 | | | | |
|---|---|---|---|---|---|---|---|
| MUTATION | PRE | POST | TOTAL | % OF TOTAL | GENE SZ | HAIR CELL | DOM/REC |
| GJB2 | 26 | 3 | 29 | 17% | 2347 | NO | BOTH |
| CDH23 | 6 | 7 | 13 | 8% | 4843 | YES | REC |
| SLC26A4 | 8 | 0 | 8 | 5% | 4930 | NO | REC |
| MYO7A | 3 | 4 | 7 | 4% | 7463 | YES | BOTH |
| OTDF | 4 | 0 | 4 | 2% | 6973 | YES | REC |
| MYO15A | 2 | 2 | 4 | 2% | 11876 | YES | REC |
| WARDNB SYN | 3 | 0 | 3 | 2% | 1504 | NO | DOM |
| TMPRSS3 | 0 | 3 | 3 | 2% | 2460 | YES | REC |
| ACTG1 | 0 | 2 | 2 | 1% | 2123 | YES | DOM |
| USHER (1=CDH23, | | | | | 4843, | | |
| 1=PCDH15) | 2 | 0 | 2 | 1% | 7042 | ? | REC |
| Mit1555A>G | 0 | 2 | 2 | 1% | h7k | ? | ? |
| GYRM | 0 | 1 | 1 | 1% | 1559 | NO | DOM |
| DFNA5 | 0 | 1 | 1 | 1% | 2276 | YES | DOM |
| COCH | 0 | 1 | 1 | 1% | 2882 | NO | DOM |
| WHRN | 0 | 1 | 1 | 1% | 2915 | YES | REC |
| LOXHD1 | 1 | 0 | 1 | 1% | 3978 | YES | REC |
| Mit3243A>G | 0 | 1 | 1 | 1% | NA | ? | ? |

The human transmembrane protease, serine 3 (TMPRSS3; also referred to as DFNB10, DFNB8, ECHOS1, TADG12; Acc: HGNC:11877) was identified by its association with both congenital (present at birth) and childhood onset autosomal recessive deafness. Mutations in the TMPRSS3 gene are associated with Autosomal Recessive Nonsyndromic Hearing Impairment type DFNB8 and 10. TMPRSS3 is a 1646 base pair gene that codes for a serine protease and is associated with DFNA 8/10 and may make up to 1-5% of patients with hearing loss undergoing cochlear implantation (Weegerink et al., 2011). Loss of function of this gene appears to result in a broad spectrum of hearing phenotypes depending on the site of the mutation. Both congenital and adult onset progressive hearing loss have been associated with the loss of this gene.

The onset of DFNB8 hearing loss is postlingual (age 10-12 years), while the onset of DFNB10 hearing loss is prelingual (congenital). This phenotypic difference reflects a genotypic difference. The DFNB8 causing variant is a splice site variant, suggesting that inefficient splicing is associated with a reduced amount of normal protein that is sufficient to prevent prelingual deafness but not sufficient to prevent eventual hearing loss. (See, Richard JH Smith, MD, et al. (2014). Genes Known to Cause Autosomal Recessive Nonsyndromic Hearing Impairment: Deafness and Hereditary Hearing Loss Overview; GeneReviews)*.*

TMPRSS3 mutations on chromosome 21 known to cause hearing loss are described in Table 2.

| **TABLE 2. TMPRSS3 MUTATIONS (CHROMOSOME 21)** | | |
|---|---|---|
| # | **MUTATION NAME** | **REFERENCE** |
| 1 | TMPRSS3, IVS4AS, G-A, -6 | Scott HS, et al. (2001) Insertion of beta-satellite repeats identifies a transmembrane protease causing both congenital and childhood onset autosomal recessive deafness. Nat Genet. 27(1):59-63. |
| 2 | TMPRSS3, 8-BP DEL, SATELLITE REPEAT INS | Scott HS, et al. (2001) Insertion of beta-satellite repeats identifies a transmembrane protease causing both congenital and childhood onset autosomal recessive deafness. Nat Genet. 27(1):59-63. |
| 3 | TMPRSS3, 1-BP DEL, 207C | Wattenhofer M, et al. (2002) Mutations in the TMPRSS3 gene are a rare cause of childhood nonsyndromic deafness in Caucasian patients. J Mol Med (Berl). 80(2):124-31. |
| 4 | c.753G>C (p.Trp251Cys) | Masmoudi S, et al. (2001) Novel missense mutations of TMPRSS3 in two consanguineous Tunisian families with non-syndromic autosomal recessive deafness. Hum Mutat. 18(2):101-8. |
| 5 | c.308A>G (p.Asp103Gly) | Wattenhofer M, et al. (2002) Mutations in the TMPRSS3 gene are a rare cause of childhood nonsyndromic deafness in Caucasian patients. J Mol Med (Berl). 80(2):124-31. |
| 6 | c.1211C>T (p.Pro404Leu) | Wattenhofer M, et al. (2005) A novel TMPRSS3 missense mutation in a DFNB8/10 family prevents proteolytic activation of the protein. Hum Genet. 117(6):528-35. |
| 7 | c.647G>T (p.Arg216Leu) | Wattenhofer M, et al. (2005) A novel TMPRSS3 missense mutation in a DFNB8/10 family prevents proteolytic activation of the protein. Hum Genet. 117(6):528-35. |
| 8 | c.579dupA (p.Cys194Metfs) | Duzkale H, et al. (2013) A systematic approach to assessing the clinical significance of genetic variants. Clin Genet. 84(5):453-63. |
| 9 | c.1192C>T (p.Gln398Ter) | Wattenhofer M, et al. (2005) A novel TMPRSS3 missense mutation in a DFNB8/10 family prevents proteolytic activation of the protein. Hum Genet. 117(6):528-35. |
| 10 | c.323-6G>A | Scott HS, et al. (2001) Insertion of beta-satellite repeats identifies a transmembrane protease causing both congenital and childhood onset autosomal recessive deafness. Nat Genet. 27(1):59-63. |
| 11 | c.916G>A (p.Ala306Thr) | Chung J, et al. (2014) A novel mutation of TMPRSS3 related to milder auditory phenotype in Korean postlingual deafness: a possible future implication for a personalized auditory rehabilitation. J Mol Med (Berl). 92(6):651-63. |
| 12 | c.208delC (p.His70Thrfs) | Battelino S, et al. (2015) TMPRSS3 mutations in autosomal recessive nonsyndromic hearing loss._Eur Arch Otorhinolaryngol. 273(5):1151-4. |
| 13 | c.1276G>A (p.Ala426Thr) | Weegerink NJ, et al. (2011) Genotype-phenotype correlation in DFNB8/10 families with TMPRSS3 mutations. J Assoc Res Otolaryngol. 12(6):753-66. |
| 14 | c.413C>A (p. Ala 13 8Glu) | Eppsteiner RW, et al. (2012) Prediction of cochlear implant performance by genetic mutation: the spiral ganglion hypothesis. Hear Res. 292(1-2):51-8. |
| 15 | c.325C>T (p.Arg109Trp) | Lee YJ, Park D, Kim SY, Park WJ (2003) Pathogenic mutations but not polymorphisms in congenital and childhood onset autosomal recessive deafness disrupt the proteolytic activity of TMPRSS3. J Med Genet. 40(8):629-31. |
| 16 | c.346G>A (p.V116M) | Ganapathy A, et al. (2014) Non-syndromic hearing impairment in India: high allelic heterogeneity among mutations in TMPRSS3, TMC1, USHIC, CDH23 and TMIE._PLoS One. 9(1):e84773. |
| 17 | c.727G>A (p.G243R) | Ganapathy A, et al. (2014) Non-syndromic hearing impairment in India: high allelic heterogeneity among mutations in TMPRSS3, TMC1, USHIC, CDH23 and TMIE._PLoS One. 9(1):e84773. |
| 18 | c.1156T>C (p.C386R) | Ganapathy A, et al. (2014) Non-syndromic hearing impairment in India: high allelic heterogeneity among mutations in TMPRSS3, TMC1, USHIC, CDH23 and TMIE._PLoS One. 9(1):e84773. |

The lipoxygenase homology domains 1 gene (LOXHD1; also referred to as LH2D1, DFNB77, FLJ32670; OMIM: 613072; Acc:HGNC:26521) encodes a highly conserved protein consisting entirely of PLAT (polycystin/lipoxygenase/alpha-toxin) domains, thought to be involved in targeting proteins to the plasma membrane. Studies in mice show that this gene is expressed in the mechanosensory hair cells in the inner ear, and mutations in this gene lead to auditory defects, indicating that this gene is essential for normal hair cell function. Screening of human families segregating deafness identified a mutation in this gene which causes DFNB77, a progressive form of autosomal-recessive nonsyndromic hearing loss (ARNSHL). Alternatively spliced transcript variants encoding different isoforms have been noted for this gene.

Clinical Features of LOXHD1:
- Autosomal recessive
- Hearing loss, sensorineural, bilateral (milder hearing loss at low frequencies)
- Congenital onset leading to cochlear implants between 7-10 years of age in Ashkenazi Jewish families
- Onset by 7-8 years of age progressing to moderate-to-severe loss of mid and high frequencies during adulthood in a consanguineous Iranian family

Evidence that autosomal recessive nonsyndromic hearing loss-77 (DFNB77) is caused by homozygous mutation in the LOXHD1 gene (613072) on chromosome 18q21.

In situ hybridization detected Loxhd1 expression in the developing mouse inner ear at embryonic days 13.5 and 16, but not in any other tissue. At postnatal day 4, expression was detected in cochlear and vestibular hair cells, with highest concentration in the nucleus. Loxhd1 progressively localized to the cytoplasm, and in the adult, Loxhd1 was expressed in hair cells along the length of stereocilia.

Using an N-ethyl-N-nitrosourea (ENU) mutagenesis screen, Grillet et al. (2009) developed the 'samba' mouse line that becomes hearing impaired by 3 weeks of age and deaf by 8 weeks of age. Homozygous samba mice showed no other neurologic or vestibular abnormalities, and heterozygous samba mice appeared completely normal. Stereociliary development was not affected in homozygous samba mice, but hair cell function was perturbed and hair cells eventually degenerated.

Grillet et al. (2009) found that samba was a mutation in the mouse Loxhd1 gene that destabilized the beta-sandwich structure of PLAT domain 10. The mutation did not alter mRNA or protein stability or localization of Loxhd1 protein along the length of stereocilia. However, by postnatal day 21, some hair cells showed morphologic defects with fused stereocilia and membrane ruffling at the apical cell surface. Profound degenerative changes were obvious by postnatal day 90, including hair cell loss and a reduction in spiral ganglion neurons. Grillet et al. (2009) hypothesized that the degeneration of spiral ganglion neurons was likely secondary to perturbations in the function and maintenance of hair cells.

LOXHD1 mutations on chromosome 18 known to cause hearing loss are described in Table 3.

| **TABLE 3. LOXHD1 MUTATIONS (CHROMOSOME 18)** | | |
|---|---|---|
| # | **MUTATION NAME** | **REFERENCE** |
| 1 | c.2008C>T (p.Arg670Ter) | Grillet N, et al. (2009) Mutations in LOXHD1, an evolutionarily conserved stereociliary protein, disrupt hair cell function in mice and cause progressive hearing loss in humans. Am J Hum Genet. 85(3):328-37. |
| 2 | c.3169C>T (p.Arg1057Ter) | Edvardson S, et al. (2011) A |
| | | deleterious mutation in the LOXHD1 gene causes autosomal recessive hearing loss in Ashkenazi Jews. Am J Med Genet A. 155A(5):1170-2. |
| | | Grillet N, et al. (2009) Mutations in LOXHD1, an evolutionarily conserved stereociliary protein, disrupt hair cell function in mice and cause progressive hearing loss in humans. Am J Hum Genet. 85(3):328-37. |
| 3 | c.2303delG (p.Gly768Alafs) | Edvardson S, et al. (2011) A |
| | | deleterious mutation in the LOXHD1 gene causes autosomal recessive hearing loss in Ashkenazi Jews. Am J Med Genet A. 155A(5):1170-2. |
| | | Grillet N, et al. (2009) Mutations in LOXHD1, an evolutionarily conserved stereociliary protein, disrupt hair cell function in mice and cause progressive hearing loss in humans. Am J Hum Genet. 85(3):328-37. |
| 4 | c.4099G>T (p.Glu1367Ter) | Edvardson S, et al. (2011) A |
| | | deleterious mutation in the LOXHD1 gene causes autosomal recessive hearing loss in Ashkenazi Jews. Am J Med Genet A. 155A(5):1170-2. |
| | | Grillet N, et al. (2009) Mutations in LOXHD1, an evolutionarily conserved stereociliary protein, disrupt hair cell function in mice and |
| | | cause progressive hearing loss in humans. Am J Hum Genet. 85(3):328-37. |
| 5 | c.2497C>T (p.Arg833Ter) | Edvardson S, et al. (2011) A |
| | | deleterious mutation in the LOXHD1 gene causes autosomal recessive hearing loss in Ashkenazi Jews. Am J Med Genet A. 155A(5):1170-2. |
| | | Grillet N, et al. (2009) Mutations in LOXHD1, an evolutionarily conserved stereociliary protein, disrupt hair cell function in mice and cause progressive hearing loss in humans. Am J Hum Genet. 85(3):328-37. |
| 6 | c.4714C>T | Edvardson S, et al. (2011) A |
| | | deleterious mutation in the LOXHD1 gene causes autosomal recessive hearing loss in Ashkenazi Jews. Am J Med Genet A. 155A(5):1170-2. |

U.S. Application Publication No. 2013/0095071, incorporated by reference herein in its entirety, describes gene therapy methods for restoring age-related hearing loss using mutated tyrosine adeno-associated viral vectors to deliver the X-linked inhibitor of apoptosis protein (XIAP) to the round window membrane of the inner ear. However, the publication does not contemplate the delivery of a nucleic acid sequence encoding functional TMPRSS3 or LOXHD 1 to prevent or delay the onset of or restore hearing loss or deafness caused by genetic mutation of the TMPRSS3 or LOXHD 1 gene, as disclosed herein.

Additionally, an important pitfall in the current state of the art for developing clinical gene therapies for hearing disorders is a lack of animal models that mirror human hearing loss. Many of the available mouse models for genetic hearing losses with adult onset in humans present with congenital hearing loss making delivery studies complex. There are few models with onset of genetic hearing loss after development of hearing. Delivery of vectors in neonatal mice results in different transfection patterns than delivery in adult mice (Shu, Tao, Li, et al., 2016). There is a need for novel animal models that can be used to evaluate rescue of hearing using different vector systems and gene targets.

There are currently no approved therapeutic treatments for preventing or treating hearing loss or deafness and there is a lack of useful preclinical animal models for testing such treatments. The present invention describes pharmaceutical compositions and methods for viral vector gene delivery of TMPRSS3 or LOXHD1 into the inner ear to restore activity of a mutated TMPRSS3 or LOXHD1 gene, promote hair cell survival and restore hearing in patients suffering from hearing loss or deafness, and animal-based models for testing such pharmaceutical compositions and methods.

### BRIEF SUMMARY

Disclosed herein is a pharmaceutical composition for use in a method for the treatment or prevention of hearing loss that includes an expression vector having the nucleic acid sequence of SEQ ID NO:1, or a nucleic acid sequence having at least 90% sequence identity to the nucleic acid of SEQ ID NO:1, wherein the nucleic acid sequence is operatively linked to a promoter. In some embodiments, the nucleic acid sequence has at least 90%, at least 95%, at least 96%, at least 97%, at least 98%, or at least 99% sequence identity to the nucleic acid sequence of SEQ ID NO:1 The expression vector is an AAV2 adeno-associated viral vector. The promoter is human cytomegalovirus (HCMV) promoter.

The references to methods of treatment in the following paragraphs of this description are to be interpreted as references to the compounds, pharmaceutical compositions and medicaments of the present invention for use in a method for treatment of the human or animal body by therapy.

Disclosed herein is a method for treating or preventing hearing loss, including administering to a subject in need thereof an effective amount of an expression vector that includes the nucleic acid sequence of SEQ ID NO:1, or a nucleic acid sequence having at least 90% sequence identity to the nucleic acid of SEQ ID NO:1, wherein the nucleic acid sequence is operatively linked to a promoter. In some embodiments, the nucleic acid sequence has at least 90%, at least 95%, at least 96%, at least 97%, at least 98%, or at least 99% sequence identity to the nucleic acid sequence of SEQ ID NO:1. The expression vector is an AAV2 adeno-associated viral vector. The promoter is human cytomegalovirus (HCMV) promoter. In some embodiments, the expression vector is administered into the inner ear of the subject, for example, by injection. In some embodiments, the delivery method is selected from cochleostomy, round window membrane, canalostomy or any combination thereof (see, Erin E. Leary Swan, et al. (2008) Inner Ear Drug Delivery for Auditory Applications. Adv Drug Deliv Rev. 60(15): 1583-1599). In some embodiments, the expression vector is delivered into the scala media via the endolymphatic sac (Colletti V, et al. (2010) Evidence of gadolinium distribution from the endolymphatic sac to the endolymphatic compartments of the human inner ear. Audiol Neurootol. 15(6):353-63; Marco Mandalà, MD, et al. (2010) Induced endolymphatic flow from the endolymphatic sac to the cochlea in Ménière's disease. Otolaryngology-Head and Neck Surgery. 143, 673-679; Yamasoba T, et al. (1999) Inner ear transgene expression after adenoviral vector inoculation in the endolymphatic sac. Hum Gene Ther. 10(5):769-74). In some embodiments, the subject has one or more genetic risk factors associated with hearing loss. In some embodiments, one of the genetic risk factors is a mutation in the TMPRSS3 gene. In some embodiments, the mutation in the TMPRSS3 gene is selected from any one or more TMPRSS3 mutations known to cause hearing loss (see, for example, Table 2). In some embodiments, one of the genetic risk factors is a mutation in the LOXHD1 gene. In some embodiments, the mutation in the LOXHD1 gene is selected from any one or more LOXHD1 mutations known to cause hearing loss (see, for example, Table 3). In some embodiments, the subject does not exhibit any clinical indicators of hearing loss.

In some embodiments, an expression vector described herein is administered as a combination therapy with one or more expression vectors comprising other nucleic acid sequences and/or with one or more other active pharmaceutical agents for treating hearing loss. For example, a combination therapy may include a first expression vector that has the nucleic acid sequence of SEQ ID NO: 1 and a second expression vector that has the nucleic acid sequence of SEQ ID NO:2, wherein both expression vectors are administered to a subject as part of a combination therapy to treat hearing loss.

Disclosed herein is a transgenic mouse having a human TMPRSS3 gene with a mutation selected from any one or more TMPRSS3 mutation known to cause hearing loss (see, for example, Table 2). Disclosed herein is a transgenic mouse having a human LOXHD1 gene with a mutation selected from any one or more LOXHD1 mutation known to cause hearing loss (see, for example, Table 3).

The present disclosure provides a pharmaceutical composition according to claim 1 and a transgenic mouse according to claim 9. The following discussion is provided for understanding the claims, but the scope of protection is only defined by the claims. In particular, the use of the word "embodiment" or "aspect" does not indicate that protection is sought beyond the scope of the claims.

### BRIEF DESCRIPTION OF THE DRAWINGS

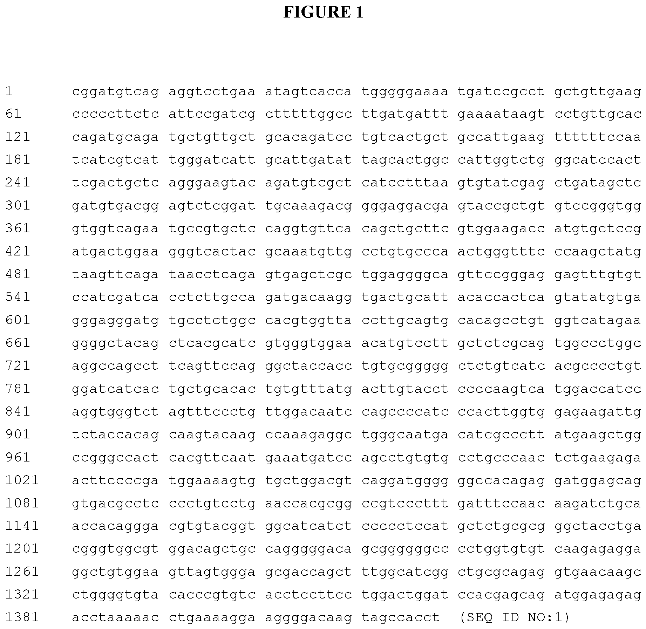

Fig. 1 shows a cDNA sequence encoding wild-type human TMPRSS3 (GenBank Accession No. BC074847.2).

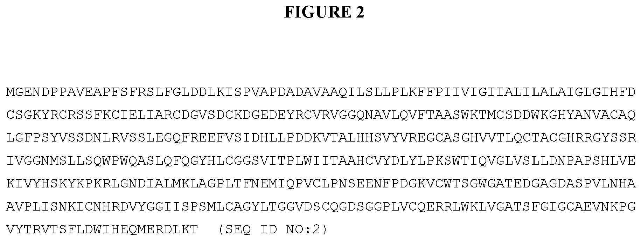

Fig. 2 shows the wild-type human TMPRSS3 amino acid sequence encoded by the cDNA in Figure 1.

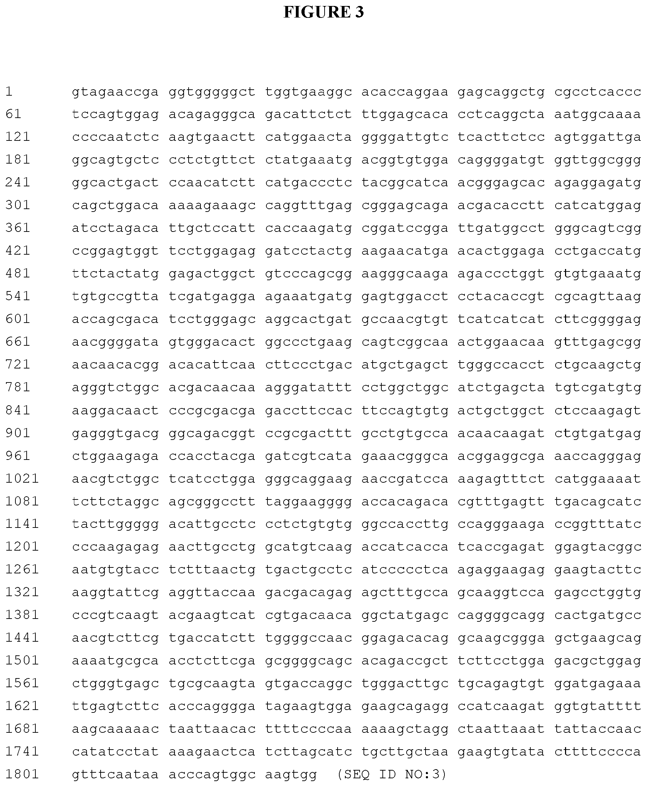

Fig. 3 shows a cDNA sequence encoding wild-type human LOXHD1 (GenBank Accession No. AK057232.1).

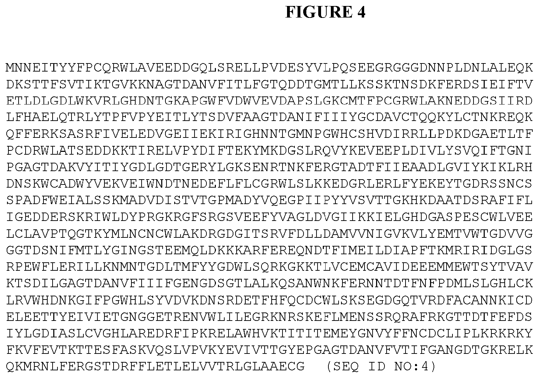

Fig. 4 shows the wild-type human LOXHD1 amino acid sequence encoded by the cDNA in Figure 3.
Fig. 5 shows Tmprss3 immunohistochemistry in the adult mouse cochlea (Fasquelle, L., Scott, H. S., Lenoir, M., Wang, J., Rebillard, G., Gaboyard, S., ... Delprat, B. (2011). Tmprss3, a transmembrane serine protease deficient in human DFNB8/10 deafness, is critical for cochlear hair cell survival at the onset of hearing. Journal of Biological Chemistry, 286(19), 17383-17397).

### DETAILED DESCRIPTION

The subject matter that is regarded as the invention is particularly pointed out and distinctly claimed in the claims at the conclusion of the specification. The foregoing and other objects, features, and advantages of the invention will be apparent from the following detailed description taken in conjunction with the accompanying drawings.

As used herein, the terms "treat," "treating," and "treatment" encompass a variety of activities aimed at desirable changes in clinical outcomes. For example, the term "treat", as used herein, encompasses any activity aimed at achieving, or that does achieve, a detectable improvement in one or more clinical indicators or symptoms of hearing loss, as described herein.

Hearing loss caused by TMPRSS3 mutations or LOXHD1 mutations generally presents in two populations: (i) the congenital population where subjects are born with hearing loss and (ii) the progressive population where subjects do not have measurable hearing loss at birth but exhibit progressive hearing loss over a period of time. Therefore, in some instances, a subject may have a mutation in the TMPRSS3 gene or in the LOXHD1 gene (for example, as detected in a genetic diagnostic test) but does not yet exhibit clinical indicators or symptoms of hearing loss, thus providing a window during which therapeutic intervention can be initiated. Accordingly, in some embodiments, the present invention provides methods for therapeutic intervention during the period of gradual regression of hearing. The methods of the present invention can be commenced prior to such time period. The methods of treating hearing loss provided by the invention include, but are not limited to, methods for preventing or delaying the onset of hearing loss or the progression of clinical indicators or symptoms of hearing loss.

As used herein, the term "hearing loss" is used to describe the reduced ability to hear sound, and includes deafness and the complete inability to hear sound.

The terms "effective amount" or "therapeutically effective amount," as used herein, refer to an amount of an active agent as described herein that is sufficient to achieve, or contribute towards achieving, one or more desirable clinical outcomes, such as those described in the "treatment" description above. An appropriate "effective" amount in any individual case may be determined using standard techniques known in the art, such as a dose escalation study.

The term "active agent" as used herein refers to a molecule (for example, an AAV vector described herein) that is intended to be used in the compositions and methods described herein and that is intended to be biologically active, for example for the purpose of treating hearing loss.

The term "pharmaceutical composition" as used herein refers to a composition comprising at least one active agent as described herein or a combination of two or more active agents, and one or more other components suitable for use in pharmaceutical delivery such as carriers, stabilizers, diluents, dispersing agents, suspending agents, thickening agents, excipients, and the like.

The terms "subject" or "patient" as used interchangeably herein encompass mammals, including, but not limited to, humans, non-human primates, rodents (such as rats, mice and guinea pigs), and the like. In some embodiments of the invention, the subject is a human.

The dose of an active agent of the invention may be calculated based on studies in humans or other mammals carried out to determine efficacy and/or effective amounts of the active agent. The dose amount and frequency or timing of administration may be determined by methods known in the art and may depend on factors such as pharmaceutical form of the active agent, route of administration, whether only one active agent is used or multiple active agents (for example, the dosage of a first active agent required may be lower when such agent is used in combination with a second active agent), and patient characteristics including age, body weight or the presence of any medical conditions affecting drug metabolism.

In one embodiment, a single dose may be administered. In another embodiment, multiple doses may be administered over a period of time, for example, at specified intervals, such as, four times per day, twice per day, once a day, weekly, monthly, and the like.

**Clinical characteristics of hearing loss.** Hereditary hearing loss and deafness may be conductive, sensorineural, or a combination of both; syndromic (associated with malformations of the external ear or other organs or with medical problems involving other organ systems) or nonsyndromic (no associated visible abnormalities of the external ear or any related medical problems); and prelingual (before language develops) or postlingual (after language develops) (Richard JH Smith, MD, et al. (2104) Deafness and Hereditary Hearing Loss Overview. GeneReviews)*.*

**Diagnosis/testing.** Genetic forms of hearing loss must be distinguished from acquired (non-genetic) causes of hearing loss. The genetic forms of hearing loss are diagnosed by otologic, audiologic, and physical examination, family history, ancillary testing (e.g., CT examination of the temporal bone), and molecular genetic testing. Molecular genetic testing, possible for many types of syndromic and nonsyndromic deafness, plays a prominent role in diagnosis and genetic counseling.

Selected tests used to measure hearing loss:
**1. Distortion Product Otoacoustic Emissions (DPOAE).** Distortion product otoacoustic emissions (DPOAE) are responses generated when the cochlea is stimulated simultaneously by two pure tone frequencies whose ratio is between 1.1 and 1.3. Recent studies on the generation mechanism of DPOAEs have underlined the presence of two important components in the DPOAE response, one generated by an intermodulation "distortion" and one generated by a "reflection".
   The prevalence of DPOAEs is 100% in normal adult ears. Responses from the left and right ears are often correlated (that is, they are very similar). For normal subjects, women have higher amplitude DPOAEs. Aging processes have an effect on DPOAE responses by lowering the DPOAE amplitude and narrowing the DPOAE response spectrum (i.e. responses at higher frequencies are gradually diminishing). The DPOAEs can be also recorded from other animal species used in clinical research such as lizards, mice, rats, guinea pigs, chinchilla, chicken, dogs and monkeys. (Otoacoustic Emissions Website).
**2. Auditory Brainstem Response (ABR).** The auditory brainstem response (ABR) test gives information about the inner ear (cochlea) and brain pathways for hearing. This test is also sometimes referred to as auditory evoked potential (AEP). The test can be used with children or others who have a difficult time with conventional behavioral methods of hearing screening. The ABR is also indicated for a person with signs, symptoms, or complaints suggesting a type of hearing loss in the brain or a brain pathway. The test is used on both humans and animals. The ABR is performed by pasting electrodes on the head-similar to electrodes placed around the heart when an electrocardiogram is run-and recording brain wave activity in response to sound. The person being tested rests quietly or sleeps while the test is performed. No response is necessary. ABR can also be used as a screening test in newborn hearing screening programs. When used as a screening test, only one intensity or loudness level is checked, and the baby either passes or fails the screen. (American Speech-Language-Hearing Association Website).

**Clinical Manifestations of hearing loss.** Hearing loss is described by type and onset:
**Type**
   - Conductive hearing loss results from abnormalities of the external ear and/or the ossicles of the middle ear.
   - Sensorineural hearing loss results from malfunction of inner ear structures (i.e., cochlea).
   - Mixed hearing loss is a combination of conductive and sensorineural hearing loss.
   - Central auditory dysfunction results from damage or dysfunction at the level of the eighth cranial nerve, auditory brain stem, or cerebral cortex.
**Onset**
   - Prelingual hearing loss is present before speech develops. All congenital (present at birth) hearing loss is prelingual, but not all prelingual hearing loss is congenital.
   - Postlingual hearing loss occurs after the development of normal speech.
   (Richard JH Smith, MD, et al.; Deafness and Hereditary Hearing Loss Overview; GeneReviews; Initial Posting: February 14, 1999; Last Revision: January 9, 2014.)

**Severity of hearing loss.** Hearing is measured in decibels (dB). The threshold or 0 dB mark for each frequency refers to the level at which normal young adults perceive a tone burst 50% of the time. Hearing is considered normal if an individual's thresholds are within 15 dB of normal thresholds. Severity of hearing loss is graded as shown in Table 4.

| **Table 4.** Severity of Hearing Loss in Decibels (dB) | |
|---|---|
| **Severity** | **Hearing Threshold in Decibels** |
| Mild | 26-40 dB |
| Moderate | 41-55 dB |
| Moderate Severe | 56-70 dB |
| Severe | 71-90 dB |
| Profound | 90 dB |

**Percent hearing impairment.** To calculate the percent hearing impairment, 25 dB is subtracted from the pure tone average of 500 Hz, 1000 Hz, 2000 Hz, 3000 Hz. The result is multiplied by 1.5 to obtain an ear-specific level. Impairment is determined by weighting the better ear five times the poorer ear (see Table 5). Because conversational speech is at approximately 50-60 dB HL (hearing level), calculating functional impairment based on pure tone averages can be misleading. For example, a 45-dB hearing loss is functionally much more significant than 30% implies. A different rating scale is appropriate for young children, for whom even limited hearing loss can have a great impact on language development [Northern & Downs 2002].

| **Table 5.** Percent Hearing Impairment | | |
|---|---|---|
| **% Impairment** | **Pure Tone Average (dB)*** | **% Residual Hearing** |
| 100% | 91 dB | 0% |
| 80% | 78 dB | 20% |
| 60% | 65 dB | 40% |
| 30% | 45 dB | 70% |
| * Pure tone average of 500 Hz, 1000 Hz, 2000 Hz, 3000 Hz | | |

**Frequency of hearing** loss. The frequency of hearing loss is designated as:
- Low (<500 Hz)
- Middle (501-2000 Hz)
- High (>2000 Hz)

**Gene therapy.** Gene therapy is when DNA is introduced into a patient to treat a genetic disease. The new DNA usually contains a functioning gene to correct the effects of a disease-causing mutation in the existing gene. Gene transfer, either for experimental or therapeutic purposes, relies upon a vector or vector system to shuttle genetic information into target cells. The vector or vector system is considered the major determinant of efficiency, specificity, host response, pharmacology, and longevity of the gene transfer reaction. Currently, the most efficient and effective way to accomplish gene transfer is through the use of vectors or vector systems based on viruses that have been made replication-defective (PCT Publication No. WO 2015/054653; Methods of Predicting Ancestral Virus Sequences and Uses Thereof).

**Vectors.** To date, adenovirus, adeno-associated virus, herpes simplex virus, vaccinia virus, retrovirus, helper dependent adenovirus and lentivirus have all tested for cochlear gene delivery. Of these, the one that has demonstrated the most potential is adeno associated virus (AAV): it is non-replicating, can efficiently transfer transgenes to the inner ear, and causes no ototoxicity. In particular, AAV can effectively transfect inner hair cells, a critical feature if one hopes to correct genetic defects due to hair cell-specific mutations. To date, a number of different AAV subtypes have been used with success for cochlear gene delivery, demonstrating little if any damage to the organ of Corti. A recent report studying AAV serotypes 1, 2, 5, 6 and 8 demonstrated successful gene expression in hair cells, supporting cells, the auditory nerve and spiral ligament, with hair cells being the most effectively transduced (Lawrence R. Lustig, MD and Omar Akil, PhD (2012) Cochlear Gene Therapy. Curr Opin Neurol. 25(1): 57-60). Examples of AAV vectors that can be administered to the inner ear are further described in U.S. Patent Application No. 2013/0095071.

The size of the target gene that can be corrected is limited based on the carrying capacity of AAV (Wu, Yang, & Colosi, 2010). For purposes of translation, this limits potential targets to those genetic disorders that are caused by relatively small genes (<4.6 kB) that cause recessive hearing loss. As an initial approach to develop a gene therapy drug, the target gene mutation should be relatively common and hearing loss should occur after the development of language. Identifying patients with progressive hearing losses that match these characteristics provides an opportunity intervene and halt or possibly reverse the progression of their loss. Inner ear gene therapy trials have started in humans for acquired deafness, therefore many of the safety and delivery issues are being addressed (Clinical trials identifier NCT02132130). The increasing availability and accuracy of genetic testing will result in the identification of patients that can benefit from these types of interventions (Preciado et al., 2005).

**Mouse models of gene therapy:** Several different mouse models of recessive genetic deafness have been rescued through gene therapy. Examples include correction of VGLUT3, TLC1, whirlin, clarin 1 mutation induced hearing losses (Akil et al., 2012; Askew et al., 2015; Chien et al., 2016; Geng et al., 2017; Isgrig et al., 2017). All of these models require delivery of the vector into the neonatal mouse inner ear before maturation of hearing and before degeneration of inner ear hair cell. This indicates that to affect rescue of hearing using a gene therapy strategy, target disorders should be at least slowly progressive in humans where to allow delivery of the therapy prior to loss of hearing and degeneration of target cells.

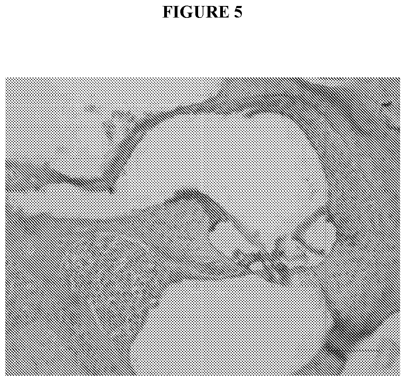

**TMPRSS3 mutations induce hearing loss:** Hearing loss related to mutations in TMPRSS3 (DFNA8/10) can present in a variety of different phenotypes. Both congenital profound hearing loss has been described as well as adult onset progressive hearing losses (Weegerink et al., 2011). Currently, the mechanism by which Tmprss3 dysfunction is unknown. Two mouse models have been developed to date hearing loss at birth and another with onset of hearing loss slightly later time point but still before the maturation of hearing and the mouse. Fasquelle et al. generated an ethyl-nitrosourea-induced mutant mouse carrying a protein-truncating nonsense mutation in Tmprss3. This demonstrated loss of hair cells and degeneration of hearing at post-natal day 12, around the time of maturation of hearing. Additionally saccular hair cells were affected and a delayed degeneration of spiral ganglion cells were noted (Fasquelle et al., 2011). It is unclear from the mouse model whether degeneration of the spiral ganglion is related to degeneration of the organ of Corti or due to dysfunction of Tmprss3 in the spiral ganglion. A number of studies have evaluated the distribution of Tmprrss3 within the mouse inner ear and largely demonstrate presence of Tmprss3 in hair cells and spiral ganglion cells (Fan, Zhu, Li, Ji, & Wang, 2014; Fasquelle et al., 2011). Expression of mouse Tmprss3 was evaluated in 1 month old C57Bl5 mice using antibody anti-TMPRSS3 (1:100, ab167160, Abcam, Cambridge, MA). Labelling was seen in inner and outer hair cells, the stria vascularis and in about 50% of spiral ganglion cells (Fig5). This suggests that loss of Tmprss3 function could additionally result in loss of strial function although no changes in endocochlear potential were seen in the Fasquelle mouse model (Fasquelle et al., 2011).

Tmprss3 genotype-phenotype studies demonstrate a wide range of different forms of hearing loss ranging from profound congenital to adult onset progressive hearing losses (Chung et al., 2014; Gao et al., 2017; Weegerink et al., 2011). Studies suggest that hearing loss due to Tmprss3 mutations may make up 2 to 5% of patients undergoing adult cochlear implantation (Jolly et al., 2012; Miyagawa, Nishio, & Usami, 2016; Sloan-Heggen et al., 2016). Many of the patients with these mutations have significant amounts of residual hearing. This would make it an attractive target for potential rescue therapy since there would be a substrate of cells that can be treated. There are some divergent studies on the success of cochlear implantation in patients with this mutation. At least some forms of hearing loss induced by loss of Tmprss3 may not do as well with cochlear implantation than other forms of genetic deafness (Shearer et al., 2017). This is potentially related to the fact that this gene is expressed both in hair cells and in up to 50% of spiral ganglion cells (see Fig. 5). These discrepancies need to be considered when choosing a vector system for delivery. Vectors will be tested with strong hair cell tropism and combined hair cell and spiral ganglion tropism. Differences in vector tropism have also been seen when comparing neonatal and adult inner ear delivery (Shu, Tao, Li, et al., 2016; Shu, Tao, Wang, et al., 2016a). Since the target clinical population are humans with a mature auditory system, disclosed herein is a mouse model that has onset of hearing loss after maturation of hearing in which can be used as a model for both disease progression (see Example 1) and model delivery of rescue therapy to the adult cochlea (see Example 2).
**AAV serotypes for delivery to the inner ear:** A wide variety of different AAV serotypes have been demonstrated to be useful in transfecting inner ear tissue (György et al., 2017; Shu, Tao, Wang, et al., 2016b; Xia, Yin, & Wang, 2012). There are clearly differences in the distribution of vector deliver transgene in neonatal and adult animals and additionally there are differences in delivery to the perilymph versus the endolymph when evaluating transfection of hair cells (Kilpatrick et al., 2011; Wang et al., 2013). Within mouse models, delivery of vector to the endolymph results in improved transfection of hair cells but results in loss of residual hearing making this approach difficult to model a progressive hearing loss. Potentially in larger animals or in humans vector could be delivered into the endolymphatic sac without causing hearing loss, thereby increasing transfection efficiency (Colletti et al., 2010). For the purposes of the planned studies, delivery into the perilymph would permit the mouse's hearing to be evaluated. AAV2 has been shown deliver to hair cells and spiral ganglion cells an adult animals (Tao et al., 2017). An additional advantage of AAV2 is that it already has an extensive track record and safety data in human gene therapy clinical trials (Santiago-Ortiz & Schaffer, 2016). Recently a variety of studies have shown that synthetic AAV (AAV2/Anc80) yields improved delivery to hair cells but may not provide equivalent delivery to the spiral ganglion as native AAV2 (Landegger et al., 2017; Suzuki, Hashimoto, Xiao, Vandenberghe, & Liberman, 2017). The invention provides a mouse model with adult onset loss of hearing and to compare whether AAV2 or AAV2/Anc80 Tmprss3 gene therapy yields better rescue of hearing and spiral ganglion function.

**Induced pluripotent stem cells (iPSCs).** An Induced Pluripotent Stem Cell (IPS or IPSCs) is a stem cell that has been created from an adult cell such as a skin, liver, stomach or other mature cell through the introduction of genes that reprogram the cell and transform it into a cell that has all the characteristics of an embryonic stem cell. The term pluripotent connotes the ability of a cell to give rise to multiple cell types, including all three embryonic lineages forming the body's organs, nervous system, skin, muscle and skeleton.

**CRISPR/Cas9 Gene Editing.** The methods described herein also contemplate the use CRISPR/Cas9 genome editing to rescue hearing by editing the TMPRSS3 gene mutation or LOXHD1 gene mutation. This technology has been used to successfully rescue hearing in two genetic hearing loss mouse models (Tmc1 and Pmca2) (Askew, C et al. (2015) Tmc gene therapy restores auditory function in deaf mice. Sci Transl Med. 7(295):295ra108). While the technology has primarily been used to target dominant hearing loss, it can be developed to target recessive hearing loss and restore hearing in the Tmprss3 knock-in mouse model, and ultimately in humans with hearing loss caused by a mutation in the TMPRSS3 gene or LOXHD1 gene. The use of CRISPR/Cas9 gene editing to repair defective gene sequences is further described in PCT Publication No. WO 2016/069910, PCT Publication No. WO 2015/048577, and U.S. Application Publication No. 2015/0291 966.

### EXAMPLES

### EXAMPLE 1 - MOUSE MODEL OF TMPRSS3 MUTATION THAT MIRRORS THE POSTNATAL ONSET PROGRESSIVE HEARING LOSS THAT SEEN IN PATIENTS

**Targeted mutation of a human TMPRSS3 mutation [c.916G>A (p.A1a306Thr)] with CRISPR/Cas9 system in the mouse:** Existing mouse models have a complete knockout of the TMPRSS3 gene that results in congenital hearing loss or a degeneration of hair cells at onset of hearing, post-natal day 12 (Fasquelle et al., 2011). This example describes the development of a knock-in mouse carrying a human TMPRSS3 mutation. The c.916G>A (p.Ala306Thr) in TMPRSS3 is the most common mutation that has been identified in more than 10 families of different ethnicities from Chinese, German, Dutch, and Korean deaf patients, suggesting that this mutation is the main contributor to the DFNB8/DFNB10 phenotype (Chung et al., 2014; Elbracht et al., 2007; Gao et al., 2017; Weegerink et al., 2011). A mouse model carrying the human mutation will be generated using CRISPR/Cas9 technique as described in detail in previous study of Harms et al. (Harms et al., 2014). Briefly, guide RNA (gRNA) design tools are used to choose appropriate gRNAs for the gene and mutation of interest; gRNA with minimal predicted off-target genomic site editing will be chosen and synthetized by a service provider, together with primers for amplifying the genomic locus of interest and other predicted off-target genomic sites.

Once established, mice will be genotyped and evaluated for hearing loss at 2, 4, and 12 weeks of age using ABR and DPOAEs. With delayed onset of hearing loss comparison of ABR and DPOAE testing may demonstrate whether reduction of Tmprss3 function asymmetrically affects hair cell or spiral ganglion function. This would affect choice of vector for clinical development given the observation in Shearer et al. (Shearer et al., 2017). Although not a prominent phenotype in humans, mouse mutants have shown subtle degeneration of saccular hair cells. Histological evaluation of the vestibular system and rotarod testing will be used to screen for balance dysfunction. Expression of mutant Tmprss3 in cochleae will be determined by quantitative RT PCR. Outcomes will be analyzed for wild type mice and homozygote Tmprss3 c.916G>A. Histological and immunohistochemical analysis of postnatal day 3, and 2, 4, and 12 week old cochlea will be carried out to identify patterns of hair cell loss. Based on histological data, elected time points will be selected for whole mount analysis of hair cell survival to determine if there are basal to apical gradients of hair cell survival. If indicated specimens may also be evaluated by scanning electron microscopy to evaluate stereociliary bundle morphology. Statistical analysis will be carried out by Graphpad Prism version 7 using ANOVA for repeated measures. Estimated animal use n=10 per time point for mutants and n=3 for controls.

Establishment of a time course of hearing loss induced by [c.916G>A (p.Ala306Thr)] will set out the time points for performing rescue studies. If successful it would also define the time course of lateral preclinical safety studies.

If the targeted mutation results in complete congenital hearing loss in the mouse, an inducible knockout mouse may be designed. Since humans show very variable phenotypes, this may also be observed in the mouse, making statistical analysis difficult. For the rescue experiments this could be addressed by using the contralateral untreated ear as a control. Alternately, the role of Tmprss3 may be evaluated in a human iPSC model (as described in Example 4). Progression of hearing loss may also be delayed in which case experimental observation will be extended to age 6 months.

### EXAMPLE 2 - EVALUATE IF HEARING LOSS AND IN THIS MOUSE MODEL CAN BE ARRESTED OR REVERSED BY TMPRSS3 GENE THERAPY

A range of vector has been proposed to deliver genes to the inner ear. For long term delivery of smaller genes, AAV vectors have been studied extensively and are safe and provide delivery to a wide range of cells. However, AAV delivery to outer hair cells has been shown to be incomplete, even at higher titers of vector. Recently a synthetic adeno associated viral vector AAV2/Anc80, has been developed that provides good to delivery to the inner and outer hair cells. Two different vector systems will be tested, one based on the potential that Tmprss3 function may also need to be rescued in the spiral ganglion. Rescue of hearing will be compared in the mouse model using these two vector systems. Potential outcomes include rescue of DPOAEs with poor ABRs in the case of AAV2/Anc80, which would indicate the need to treat the spiral ganglion in addition to hair cells. In brief, vector will be delivered to a canalostomy of the posterior semicircular canal at one to two weeks prior to documented onset of hearing loss based on results from Example 1. Three different concentrations of vector at log fold differences will be injected using a micro pump. Hearing in the animals will be evaluated with serial ABRs and DPOAEs at time points determined in Example 1. At 3 to 6 months of age animals will be evaluated for hair cell survival by histology, immunohistochemistry and whole mount evaluation of the cochlea. Controls will consist of animals treated with GFP only expressing vectors (AAV2.hCMV.GFP or AAV2/Anc80.hCMV.GFP). If mice additionally demonstrate any vestibular they will also be evaluated by the vestibular system histology and rotarod testing. Additionally, toxicity of Tmprss3 therapy will be assessed by transfecting wild type mice with both vectors expressing Tmprss3. Statistical analysis will be carried out by Graphpad Prism version 7 using ANOVA for repeated measures. Outcomes will be evaluated based on hearing per vector titer. Estimated animal use n=60 per time point (2 vectors, 3 concentrations of vector; histology and whole mount evaluation) for mutants and n=3 for controls. Overexpression studies will use n=5 animals with 2 vectors.

Tmprss3 gene therapy is expected to prevent progression of hearing loss. There may be differences in the different vectors to rescue hearing as well as dosage effects.

If rescue of hearing is found to be incomplete, different vectors may be designed. Potentially expression of Tmprss3 may need to be optimized by using different strength promoters or including the regulatory regions of Tmprss3. Potentially Tmprss3 gene therapy may have to be provided to the inner ear at a very early time point.

**Evaluation of an AAV2 TMPRSS3 delivery system:** Human TMPRSS3 (BC074847) driven by the human CMV (HCMV) promoter was cloned in to an AAV2 vector system. Vector was purified over a cesium chloride gradient. Titers were determined by pPCR (1.1 × 10¹³ GC/ml). After synthesis and purification, vector was aliquoted in 20 µl volumes and stored in PBS in 5% glycerol at -80°C.

**Development of an assay system for TMPRSS3 delivery:** In order to evaluate the efficiency of vector function we wanted to develop a system for assaying TMPSS3 production. This would also serve to evaluate later vector designs with different promoters and to evaluate vector stability. Cell lines do have differences in transfection efficiency with different AAV serotypes (Ellis et al., 2013). A variety of cell lines were screened for ability to be transfected with AAV2. Unfortunately, many of the commonly used cell lines that are transfectable with AAV2 express native TMPRSS3. A variety of human cancer cell lines were screened that have been confirmed by RNAseq not to express TMPRSS3. Primary Juvenile nasopharyngeal angiofibroma (JNA) cells were found to transfect with AAV2hcmv.gfp. Cells were plated in T-75 flasks supplemented with DMEM and 10% heat inactivated fetal bovine serum (FBS) (Invitrogen, Carlsbad, CA) while being maintained at 37°C and 5% CO₂. Media changes occurred every 48 hours. When cells reached P4 and were 90-100% confluent, 3ml of 0.05% Trypsin was added to wash, aspirated then added 3ml of Trypsin to remove cells. Incubation occurred at 37 degrees for 3-5 minutes. Trypsin was neutralized with 10%FBS/DMEM. Solution of cells were collected in a 15ml conical vial and spun at 1200 rpm for 5 minutes at 4 degrees. Cells were then suspended in 10ml of 10% FBS/DMEM and plated, allowing 24 hours to adhere. Transduction of AAV2-TMPRSS3 was performed by plating cells on 4 well chambered Millicell EZ Slides (PEZGS0416, MilliporeSigma, Burlington, MA) seeding cells with 2ul/ml concentration of AAV2.hcmv.TMPRSS3 in 500µL of medium. Cell media was changed after 24 hours then every 48 hours post transfection until cells were analyzed.

**Immunocytochemistry of AAV2-TMPRSS3 transfected Cells:** Transfected JNA cells were initially washed with PBS then fixed with 4% paraformaldehyde in PBS at room temperature for 10 minutes. Cells were blocked and permeabilized with 1% Triton - X 100 / 1% bovine serum albumin / 10% normal serum for one hour at room temperature. Primary antibody (1:100, ab 167160, Abcam, Cambridge, MA/ 1:50, GTX81644, GeneTex, Irvine, CA) was incubated overnight at 4°C. The following day, cells were rinsed with PBS then labeled with Alexa Fluor 488 (1:500, Invitrogen, Carlsbad, CA). These cells were further washed with PBS then stained using ProLong Gold antifade mounting medium (Invitrogen, Carlsbad, CA) with 0.2pg/ml 4',6-diamidino-2-phenylindole.

**Construction of AAV/Anc80-TMPRSS3-IRES-GFP.** We will clone a human TMPRSS3 full length cDNA into AAV/Anc80 vector with a eGFP marker so cells infected with the virus can be tracked by GFP expression. We have obtained a human TMPRSS3 full length cDNA with the sequence verified. We will use the AAV/Anc80 vector for cloning. Previously a human ISL1 gene was cloned into the same vector and injected AAV/Anc80-ISL1-IRES-GFP into neonatal and adult mouse inner ears by cochleostomy and canalostomy, respectively. AAV/Anc80 mediates gene delivery into neonatal and adult hair cells efficiently, without causing hair cell damage or hearing loss. Further expression of the transgene is maintained over time, which is ideal for our goal to express TMPRSS3 gene in the Tmprss3^{-/-} hair cells to restore gene expression and to recover hearing (Shu et al., 2016; Tao et al., 2017).

After cloning of TMPRSS3 into the vector of AAV/Anc80-TMPRSS3-IRES-GFP, the vector will be amplified and packaged, with the aim to achieve a viral titer of 10¹². We will first test the AAV/Anc80-TMPRSS3-IRES-GFP virus by infecting human cells. We will perform immunolabeling with an anti-TMPRSS3 antibody and co-localize it with GFP, to examine if TMPRSS3 and GFP expressed in the same cells. For *in vivo* study, we will test the vector by injecting into wildtype neonatal inner ear by cochleostomy and adult inner ear by canalostomy, respectively. Two weeks after injection, mouse cochleae will be harvested to examine if the GFP signals are confined to hair cells. In our previous studies, we observed strong GFP expression in 100% inner hair cells and moderated expression in over 95% of outer hair cells. We thus expect to observe a similar expression pattern with the TMPRSS3 gene.

AAV/Anc80-TMPRSS3-GFP will be subsequently injected into Tmprss3^{-/-} cochlea by cochleostomy with hearing studied one month later. We expect to see better hearing in the injected inner ears compared with uninjected control inner ears. This will be evident especially if Tmprss3^{-/-} mice suffer from profound early onset hearing loss. If hearing loss in the Tmprss3^{-/-} is progressive with delayed onset, we may start to see hearing rescue at later stage, e.g. 2 and 3 months of age. Hearing studies will be carried by ABR and DPOAE. If ABR and DPOAE thresholds are significantly lower at any frequency in the injected ears, it is an indication of AAV-mediated hearing rescue in Tmprss3^{-/-} mice. We will follow the progression of hearing rescue for 6 months, to determine if hearing recovery is sustained.

In Tmprss3^{-/-} mice, hair cells will eventually die. With AAV-mediated gene delivery, recovery in hair cell function will likely lead to hair cell survival over time, which will be confirmed by hair cell counting along the length of cochlea over time. We expect to see significantly more hair cells in the injected cochleae compared with uninjected control cochleae. Comparison of hearing outcomes and cell survival will determine which is the optimal vector and dose of vector for rescue therapy.

### EXAMPLE 3 - TMPRSS3

**DEVELOPMENT OF A TMPRSS3-MUTANT MOUSE MODEL.** The development of a mouse model that resembles human condition as close as possible is a key to future therapy. A knock-out Tmprss3 mouse model is available from commercial vendors and may be used in the experiments described in these Examples, however, it will be more relevant to develop a mouse model that harbors a human mutation known to cause hearing loss, as described above. The model is expected to show that the human mutation causes hearing loss in mouse as in human, which makes the model valuable to be studied for treatment. Methods for producing transgenic mouse lines are used routinely in the art and would be known to one skilled in the art. Importantly, the time to generate a mouse model has been shortened significantly, from an average of 2 years just a few years ago to a few months these days with the use of CRISPR/Cas9 technology. Thus the time it takes to produce a mouse model is no longer a rate-limiting factor. We are proposing to create a TMPRSS3 mouse model carrying a human mutation for our study. In TMPRSS3 knock-out mice, hair cells die and mice exhibit profound hearing loss. After creation of Tmprss3 knock-in mouse model, we will study survival of hair cells and hearing loss by ABR and DPOAE. If the mouse model exhibits progressive hearing loss of the loss of hair cells, it is the demonstration that we have generated Tmprss3 mouse model for human DFNB8.

**TO PRODUCE AAV-TMPRSS3 FOR GENE THERAPY.** We will use AAV-mediated gene therapy to treat Tmprss3 mutant mice. We have identified two AAV vectors (Anc80 and AAV2) for the study, but other expression vectors may also be tested, such as an adenoviral vector, a herpes simplex viral vector, a vaccinia viral vector, a helper dependent adenoviral vector, a lentiviral vector or other adeno-associated viral vectors such as AAV5, AAV6, AAV6.2, AAV7, AAV8, AAV9, AAVrh8, AA Vrh10, AAVrh39, AAVrh43, AAV1, AAV2, AAV3, AAV4, AAV5, AAV6, AAV7 or AAV8. Both Anc80 and AAV2 have high efficiency to deliver genes into mammalian neonatal hair cells without adversely affecting normal hearing. Further it was recently shown that both can be used to deliver genes into adult mouse hair cells without affecting normal hearing (Zinn, E. et al., In Silico Reconstruction of the Viral Evolutionary Lineage Yields a Potent Gene Therapy Vector, Cell Rep. 2015 Aug 11;12(6): 1056-68; and Askew, C. et al., Tmc gene therapy restores auditory function in deaf mice, Sci Transl Med. 2015 Jul 8;7(295):295ra108). We will clone a human TMPRSS3 cDNA into a vector to produce AAV- TMPRSS3 virus. We will perform study to show that TMPRSS3 is produced using in vitro culture system. Following the confirmation, the AAV-TMPRSS3 will be injected into neonatal wildtype mouse inner ear to show it does not have adverse effect, shown by cellular analysis of inner ear and by hearing study (ABR and DPOAE).

**STUDY OF AAV-TMPRSS3 IN RESTORATION OF HEARING.** We will inject AAV-TMPRSS3 into the neonatal Tmprss3 knock-in mutant mouse inner ear. Analysis will be performed for the injected and control mice injected with AAV-GFP, which will include hearing test, cellular and molecular studies and long-term effect. At cellular level we will determine if AAV-TMPRSS3 promotes hair cell survival at one month of age. In control mutant ears injected with AAV-GFP, we expect to see the loss of hair cells at this time point. In contrast we expect to see AAV-TMPRSS3 injected hair cells survive. We will optimize the injection procedure (cochleostomy, round window membrane, canalostomy) and doses for better hearing recovery. Importantly we will perform injection in adult (1-6 months of age) mice and assess hearing recovery. Adult injection results will be compared with neonatal results, which will inform us about the time window in which intervention is still effective.

### EXAMPLE 4 - STUDY OF HAIR CELLS DERIVED FROM PATIENT INDUCED PLURIPOTENT STEM CELLS (iPS) CELLS

One important aspect of the study is to demonstrate our strategy works with human hair cells. As no human temporal bone is available for the study, we will instead establish patient iPS cell lines using patient fibroblasts as well as control family member fibroblasts. The fibroblast will be harvested from the patients with the most frequent mutation and the iPS cell lines will be established. The iPS cell lines will be differentiated into inner ear cells including hair cells. With the culture system, AAV-TMPRSS3 will be used to infect iPS-derived hair cells. Infected hair cells will be studied for survival and hair cell transduction by patchy clamping. We expect to see improved hair cell survival and hair cell function, compared to the uninfected and un-treated control hair cells. The study will provide the opportunities to evaluate the efficiency of AAV-TMPRSS3 infection in human hair cells and expression of TMPRSS3 gene. Such achievement is a demonstration that defective human hair cells can be treated with AAV-TMPRSS3, which makes it one major step forward to future clinical studies.

### EXAMPLE 5 - LOXHD1

**DEVELOPMENT OF A LOXHD1-MUTANT MOUSE MODEL.** The development of a mouse model that resembles human condition as close as possible is a key to future therapy. A knock-out LOXHD1 mouse model will be relevant to develop a model that harbors a human mutation known to cause hearing loss, as described above. The model is expected to show that the human mutation causes hearing loss in mouse as in human, which makes the model valuable to be studied for treatment. Methods for producing transgenic mouse lines are used routinely in the art and would be known to one skilled in the art. Importantly, the time to generate a mouse model has been shortened significantly, from an average of 2 years just a few years ago to a few months these days with the use of CRISPR/Cas9 technology. Thus the time it takes to produce a mouse model is no longer a rate-limiting factor. We are proposing to create a LOXHD1 mouse model carrying a human mutation for our study. In LOXHD1 knock-out mice, hair cells die and mice exhibit profound hearing loss. After creation of LOXHD1 knock-in mouse model, we will study survival of hair cells and hearing loss by ABR and DPOAE. If the mouse model exhibits progressive hearing loss of the loss of hair cells, it is the demonstration that we have generated LOXHD1 mouse model for human DFNB77.

**TO PRODUCE AAV-LOXHD1 FOR GENE THERAPY.** We will use AAV-mediated gene therapy to treat LOXHD1 mutant mice. We have identified two AAV vectors (Anc80 and AAV2) for the study, but other expression vectors may also be tested, such as an adenoviral vector, a herpes simplex viral vector, a vaccinia viral vector, a helper dependent adenoviral vector, a lentiviral vector or other adeno-associated viral vectors such as AAV5, AAV6, AAV6.2, AAV7, AAV8, AAV9, AAVrh8, AA Vrh10, AAVrh39, AAVrh43, AAV1, AAV2, AAV3, AAV4, AAV5, AAV6, AAV7 or AAV8. Both Anc80 and AAV2 have high efficiency to deliver genes into mammalian neonatal hair cells without adversely affecting normal hearing. Further it was recently shown that both can be used to deliver genes into adult mouse hair cells without affecting normal hearing (Zinn, E. et al. (2015) In Silico Reconstruction of the Viral Evolutionary Lineage Yields a Potent Gene Therapy Vector. Cell Rep. 11;12(6):1056-68; and Askew, C. et al. (2015) Tmc gene therapy restores auditory function in deaf mic. Sci Transl Med. 7(295):295ra108). We will clone a human LOXHD1 cDNA into a vector to produce AAV- LOXHD1 virus. We will perform study to show that LOXHD1 is produced using in vitro culture system. Following the confirmation, the AAV-LOXHD1 will be injected into neonatal wildtype mouse inner ear to show it does not have adverse effect, shown by cellular analysis of inner ear and by hearing study (ABR and DPOAE).

**STUDY OF AAV-LOXHD1 IN RESTORATION OF HEARING.** We will inject AAV-LOXHD1 into the neonatal LOXHD1 knock-in mutant mouse inner ear. Analysis will be performed for the injected and control mice injected with AAV-GFP, which will include hearing test, cellular and molecular studies and long-term effect. At cellular level we will determine if AAV-LOXHD1 promotes hair cell survival at one month of age. In control mutant ears injected with AAV-GFP, we expect to see the loss of hair cells at this time point. In contrast we expect to see AA V-LOXHD1 injected hair cells survive. We will optimize the injection procedure (cochleostomy, round window membrane, canalostomy) and doses for better hearing recovery. Importantly we will perform injection in adult (1-6 months of age) mice and assess hearing recovery. Adult injection results will be compared with neonatal results, which will inform us about the time window in which intervention is still effective.

**STUDY OF HAIR CELLS DERIVED FROM PATIENT INDUCED PLURIPOTENT STEM CELLS (iPS) CELLS.** One important aspect of the study is to demonstrate our strategy works with human hair cells. As no human temporal bone is available for the study, we will instead establish patient iPS cell lines using patient fibroblasts as well as control family member fibroblasts. The fibroblast will be harvested from the patients with the most frequent mutation and the iPS cell lines will be established. The iPS cell lines will be differentiated into inner ear cells including hair cells. With the culture system, AAV-LOXHD1 will be used to infect iPS-derived hair cells. Infected hair cells will be studied for survival and hair cell transduction by patchy clamping. We expect to see improved hair cell survival and hair cell function, compared to the uninfected and un- treated control hair cells. The study will provide the opportunities to evaluate the efficiency of AAV- LOXHD1 infection in human hair cells and expression of LOXHD1 gene. Such achievement is a demonstration that defective human hair cells can be treated with AAV-LOXHD1, which makes it one major step forward to future clinical studies.

### EXAMPLE 6 - EXEMPLARY METHODS

**ABR Measurement:** ABR thresholds were recorded using the Intelligent Hearing Systems Smart EP program (IHS, Miami, FL, U.S.A.). Animals were anesthetized as described above and kept warm on a heating pad (37°C). Needle electrodes were placed on the vertex (+), behind the left ear (-) and behind the opposite ear (ground). Tone bursts were presented at 4, 8, 16 and 32 kHz, with duration of 500 µs using a high frequency transducer. Recording was carried out using a total gain equal to 100K and using 100 Hz and 15 kHz settings for the high and low-pass filters. A minimum of 128 sweeps was presented at 90 dB SPL. The SPL was decreased in 10 dB steps. Near the threshold level, 5 dB SPL steps using up to 1024 presentations were carried out at each frequency. Threshold was defined as the SPL at which at least one of the waves could be identified in 2 or more repetitions of the recording. The preoperative threshold was measured prior to the first operation and the final postoperative threshold was measured before sacrificing the animals. We tested mice prior each vector delivery and three days post final vector delivery.

**DPOAE Measurement:** To evaluate the functional damage on the OHC, distortion product otoacoustic emissions (DPOAE) and the input/output function (I/O Function) were recorded on both sides using the IHS Program described above. The distortion products were measured for pure tones from 2 kHz to 32 kHz using the IHS high frequency transducer. The Etymotic 10B+ Probe was inserted to the external ear canal. L1 Level was set to 65 dB L2 Level was set to 55 dB. Frequencies were acquired with an F2-F1 ratio of 1.22 using 16 sweeps. I/O Functions were acquired at the frequency of 16 kHz. Nine stimulus levels ranging from 65 dB SPL to 31 dB SPL were used in 5 dB steps. Mice were tested prior each vector delivery and three days post final vector delivery.

**Delivery of TMPRSS3 expressing AAV to the inner ear:** All procedures were reviewed and approved by an appropriate Animal Care and Use Committee. Adult mice were anesthetized with an i. p. injection of a mixture of ketamine (150 mg/kg), xylocaine (6 mg/kg) and acepromazine (2 mg/kg) in sodium chloride 0.9%. The vector was either delivered to the posterior semicircular canal. A dorsal postauricular incision was made, and the posterior or lateral semicircular canal exposed. Using a microdrill, a canalostomy was created, exposing the perilymphatic space. Subsequently 0.5 µl of vector was injected using a Hamilton microsyringe with 0.1 µl graduations and a 36 gauge needle. The canalostomy will be sealed with bone wax, and the animals were allowed to recover.

**Protocol for Rotarod (RR) training and testing of mice:** For training, mice are placed on the RR Rotarod (ENV-575M, Med Associated Inc., Georgia, USA), one at a time and program is initialized at a rate of 4-40rpm. As the mouse falls/jumps/stops and rotates in the first slot, they are picked up and placed into the consecutive slot without stopping the program. After treatment, mice are placed on RR, one mouse in each slot and program is initiated. As a mouse falls off, they are picked up and placed in animal housing unit to wait for the other mice to finish the program.

### REFERENCES

Akil, O., Seal, R. P., Burke, K., Wang, C., Alemi, A., During, M., ... Lustig, L. R. (2012). Restoration of Hearing in the VGLUT3 Knockout Mouse Using Virally Mediated Gene Therapy. Neuron, 75(2), 283-293.
Askew, C., Rochat, C., Pan, B., Asai, Y., Ahmed, H., Child, E., ... Holt, J. R. (2015). Tmc gene therapy restores auditory function in deaf mice. Science Translational Medicine, 7(295), 295ra108.
Chien, W. W., Isgrig, K., Roy, S., Belyantseva, I. A., Drummond, M. C., May, L. A., ... Cunningham, L. L. (2016). Gene Therapy Restores Hair Cell Stereocilia Morphology in Inner Ears of Deaf Whirler Mice. Molecular Therapy, 24(1), 17-25.
Chung, J., Park, S. M., Chang, S. O., Chung, T., Lee, K. Y., Kim, A. R., ... Choi, B. Y. (2014). A novel mutation of TMPRSS3 related to milder auditory phenotype in Korean postlingual deafness: a possible future implication for a personalized auditory rehabilitation. Journal of Molecular Medicine, 92(6), 651-663.
Colletti, V., Mandalà, M., Carner, M., Barillari, M., Cerini, R., Pozzi Mucelli, R., & Colletti, L. (2010). Evidence of gadolinium distribution from the endolymphatic sac to the endolymphatic compartments of the human inner ear. Audiology & Neuro-Otology, 15(6), 353-63.
Elbracht, M., Senderek, J., Eggermann, T., Thürmer, C., Park, J., Westhofen, M., & Zerres, K. (2007). Autosomal recessive postlingual hearing loss (DFNB8): compound heterozygosity for two novel TMPRSS3 mutations in German siblings. Journal of Medical Genetics, 44(6), e81.
Ellis, B. L., Hirsch, M. L., Barker, J. C., Connelly, J. P., Steininger, R. J., & Porteus, M. H. (2013). A survey of ex vivo/in vitro transduction efficiency of mammalian primary cells and cell lines with Nine natural adeno-associated virus (AAV1-9) and one engineered adeno-associated virus serotype. Virology Journal, 10(1), 74.
Fan, D., Zhu, W., Li, D., Ji, D., & Wang, P. (2014). Identification of a novel homozygous mutation, TMPRSS3: C.535G>A, in a tibetan family with autosomal recessive non-syndromic hearing loss. PLoS ONE, 9(12), 1-13.
Fasquelle, L., Scott, H. S., Lenoir, M., Wang, J., Rebillard, G., Gaboyard, S., ... Delprat, B. (2011). Tmprss3, a transmembrane serine protease deficient in human DFNB8/10 deafness, is critical for cochlear hair cell survival at the onset of hearing. Journal of Biological Chemistry, 286(19), 17383-17397.
Gao, X., Yuan, Y. Y., Wang, G. J., Xu, J. C., Su, Y., Lin, X., & Dai, P. (2017). Novel mutations and mutation combinations of TMPRSS3 cause various phenotypes in one Chinese family with autosomal recessive hearing impairment. BioMed Research International, 2017*.*
Geng, R., Omar, A., Gopal, S. R., Chen, D. H.-C., Stepanyan, R., Basch, M. L., ... Alagramam, K. N. (2017). Modeling and Preventing Progressive Hearing Loss in Usher Syndrome III. Scientific Reports, 7(1), 13480.
György, B., Sage, C., Indzhykulian, A. A., Scheffer, D. I., Brisson, A. R., Tan, S., ... Maguire, C. A. (2017). Rescue of Hearing by Gene Delivery to Inner-Ear Hair Cells Using Exosome-Associated AAV. Molecular Therapy, 25(2), 379-391.
Harms, D. W., Quadros, R. M., Seruggia, D., Ohtsuka, M., Takahashi, G., Montoliu, L., ... Gurumurthy, C. B. (2014). Mouse Genome Editing Using the CRISPR/Cas System. In Current Protocols in Human Genetics (p. 15.7.1-15.7.27). Hoboken, NJ, USA: John Wiley & Sons, Inc.
Isgrig, K., Shteamer, J. W., Belyantseva, I. A., Drummond, M. C., Fitzgerald, T. S., Vijayakumar, S., ... Chien, W. W. (2017). Gene Therapy Restores Balance and Auditory Functions in a Mouse Model of Usher Syndrome. Molecular Therapy, 25(3), 780-791.
Jolly, C., Mistrik, P., Rajan, G., Green, K., Staecker, H., Helbig, S., & Usami, S.-I. (2012). New trends with cochlear implants and atraumaticity demonstrated by deep electrode insertion and hearing preservation. Practica Oto-Rhino-Laryngologica, (SUPPL. 132).
Kilpatrick, L. A., Li, Q., Yang, J., Goddard, J. C., Fekete, D. M., & Lang, H. (2011). Adeno-associated virus-mediated gene delivery into the scala media of the normal and deafened adult mouse ear. Gene Therapy, 18(6), 569-578.
Landegger, L. D., Pan, B., Askew, C., Wassmer, S. J., Gluck, S. D., Galvin, A., ... Vandenberghe, L. H. (2017). A synthetic AAV vector enables safe and efficient gene transfer to the mammalian inner ear. Nature Biotechnology, 35(3), 280-284.
Miyagawa, M., Nishio, S.-Y., & Usami, S.-I. (2016). A Comprehensive Study on the Etiology of Patients Receiving Cochlear Implantation With Special Emphasis on Genetic Epidemiology. Otology & Neurotology, 37(2), e126-e134.
Preciado, D. a, Lawson, L., Madden, C., Myer, D., Ngo, C., Bradshaw, J. K., ... Greinwald, J. H. (2005). Improved diagnostic effectiveness with a sequential diagnostic paradigm in idiopathic pediatric sensorineural hearing loss. Otology & Neurotology : Official Publication of the American Otological Society, American Neurotology Society [and] European Academy of Otology and Neurotology, 26(4), 610-5.
Santiago-Ortiz, J. L., & Schaffer, D. V. (2016). Adeno-associated virus (AAV) vectors in cancer gene therapy.
Shearer, A. E., Eppsteiner, R. W., Frees, K., Tejani, V., Sloan-Heggen, C. M., Brown, C., ... Smith, R. J. H. (2017). Genetic variants in the peripheral auditory system significantly affect adult cochlear implant performance. Hearing Research, 348, 138-142.
Shu, Y., Tao, Y., Li, W., Shen, J., Wang, Z., & Chen, Z.-Y. (2016). Adenovirus Vectors Target Several Cell Subtypes of Mammalian Inner Ear In Vivo. Neural Plasticity, 2016, 1-8.
Shu, Y., Tao, Y., Wang, Z., Tang, Y., Li, H., Dai, P., ... Chen, Z.-Y. (2016a). Identification of Adeno-Associated Viral Vectors That Target Neonatal and Adult Mammalian Inner Ear Cell Subtypes. Human Gene Therapy, 27(9), 687-699.
Shu, Y., Tao, Y., Wang, Z., Tang, Y., Li, H., Dai, P., ... Chen, Z.-Y. (2016b). Identification of Adeno-Associated Viral Vectors That Target Neonatal and Adult Mammalian Inner Ear Cell Subtypes. Human Gene Therapy, 27(9), 687-699.
Sloan-Heggen, C. M., Bierer, A. O., Shearer, A. E., Kolbe, D. L., Nishimura, C. J., Frees, K. L., ... Smith, R. J. H. (2016). Comprehensive genetic testing in the clinical evaluation of 1119 patients with hearing loss. Human Genetics, 135(4), 441-450.
Suzuki, J., Hashimoto, K., Xiao, R., Vandenberghe, L. H., & Liberman, M. C. (2017). Cochlear gene therapy with ancestral AAV in adult mice: complete transduction of inner hair cells without cochlear dysfunction. Scientific Reports, 7, 45524.
Tao, Y., Huang, M., Shu, Y., Ruprecht, A., Wang, H., Tang, Y., ... Chen, Z.-Y. (2017). Delivery of Adeno-Associated Viral Vectors in Adult Mammalian Inner Ear Cell Subtypes without Auditory Dysfunction. Human Gene Therapy, hum.2017.120.
Wang, Y., Sun, Y., Chang, Q., Ahmad, S., Zhou, B., Kim, Y., ... Lin, X. (2013). Early postnatal virus inoculation into the scala media achieved extensive expression of exogenous green fluorescent protein in the inner ear and preserved auditory brainstem response thresholds. The Journal of Gene Medicine, 15(3-4), 123-133.
Weegerink, N. J. D., Schraders, M., Oostrik, J., Huygen, P. L. M., Strom, T. M., Granneman, S., ... Kunst, H. P. M. (2011). Genotype-phenotype correlation in DFNB8/10 families with TMPRSS3 mutations. JARO - Journal of the Association for Research in Otolaryngology, 12(6), 753-766.
Wu, Z., Yang, H., & Colosi, P. (2010). Effect of genome size on AAV vector packaging. Molecular Therapy : The Journal of the American Society of Gene Therapy, 18(1), 80-6.
Xia, L., Yin, S., & Wang, J. (2012). Inner Ear Gene Transfection in Neonatal Mice Using Adeno-Associated Viral Vector: A Comparison of Two Approaches. PLoS ONE, 7(8), e43218.

One or more embodiments of the present invention have been described. Nevertheless, it will be understood that various modifications may be made without departing from the scope of the invention as defined by the appended claims.

## Claims

1. A pharmaceutical composition for use in a method for the treatment or prevention of hearing loss in a subject in need thereof, comprising:
an adeno-associated viral vector selected to be AAV2 having a human TMPRSS3 nucleic acid sequence selected from the group consisting of SEQ ID NO: 1 and a nucleic acid sequence having at least 90% sequence identity to the nucleic acid of SEQ ID NO: 1, and a human cytomegalovirus (HCMV) promoter operatively linked to the nucleic acid.

2. The pharmaceutical composition for use according to claim 1, wherein the viral vector titer is 1.1 × 10¹³ genome copies per milliliter (GC/mL), as determined by pPCR.

3. The pharmaceutical composition for use according to claim 1 or 2, the method comprising:
administering to the subject in need thereof an amount of an adeno-associated viral vector selected to be AAV2 and having a human TMPRSS3 nucleic acid sequence selected from the group consisting of SEQ ID NO: 1, and a nucleic acid sequence having at least 90% sequence identity to the nucleic acid of SEQ ID NO: 1, and a human cytomegalovirus (HCMV) promoter operatively linked to the nucleic acid.

4. The pharmaceutical composition for use according to claim 3 , wherein the effective amount is effective to restore gene expression at hair cells in the subject's ear.

5. The pharmaceutical composition for use according to any one of claim 3 or claim 4, wherein the adeno-associated viral vector is administered by an injection method, and wherein the injection method is selected from the group consisting of cochleostomy, injection into the round window membrane, injection into the endolymphatic sac, injection into the scala media, canalostomy, injection into the scala media via the endolymphatic sac, or any combination thereof.

6. The pharmaceutical composition for use according to claim 3, wherein the subject has one or more genetic risk factors associated with hearing loss.

7. The pharmaceutical composition for use according to claim 6, wherein one of the genetic risk factors is selected from the group consisting of a mutation in the TMPRSS3 gene or a mutation in the LOXHD1 gene.

8. The pharmaceutical composition for use according to claim 6, wherein the subject does not exhibit any clinical indicators of hearing loss.

9. A transgenic mouse designed to have one or more knock-in mutations that cause hearing loss, the mutations each corresponding to a mutation in a human TMPRSS3 known to cause hearing loss, wherein the one or more knock-in mutations are selected from the group consisting of: IVS4AS, G-A, -6; 8-BP DEL, SATELLITE REPEAT INS; 1-BP DEL 207C; c.753G>C (p.Trp251Cys); c.308A>G (p.Asp103Gly); c.1211C>T (p.Pro404Leu); c.647G>T (p.Arg216Leu); c.579dupA (p.Cys194Metfs); c.1192C>T (p.Gln398Ter); c.323-6G>A; c.916G>A (p.Ala306Thr); c.208delC (p.His70Thrfs); c.1276G>A (p.Ala426Thr); c.413C>A (p.Ala138Glu); c.325C>T (p.Arg109Trp); c.346G>A(p.V116M); c.727G>A (p.G243R); and c.1156T>C (p.C386R).

10. The transgenic mouse of claim 9. wherein the mutation is c.916G>A (p.Ala306Thr).

## Patentansprüche

1. Pharmazeutische Zusammensetzung zur Verwendung in einem Verfahren zur Behandlung oder Vorbeugung von Hörverlust bei einem Individuum, das hierfür einen Bedarf hat, umfassend:
einen Adeno-assoziierten viralen Vektor, der ausgewählt ist, AAV2 zu sein, mit einer humanen TMPRSS3-Nukleinsäuresequenz, welche ausgewählt ist aus der Gruppe bestehend aus SEQ ID NO:1 und einer Nukleinsäuresequenz, die mindestens 90% Sequenzidentität mit der Nukleinsäuresequenz von SEQ ID NO:1 aufweist, und einem Humanen Cytomegalievirus (HCMV)-Promotor, der operativ mit der Nukleinsäure gekoppelt ist.

2. Pharmazeutische Zusammensetzung zur Verwendung nach Anspruch 1, wobei der Titer des viralen Vektors 1,1 × 10¹³ Genomkopien pro Milliliter (GC/mL) beträgt, wie durch pPCR bestimmt.

3. Pharmazeutische Zusammensetzung zur Verwendung nach Anspruch 1 oder 2, wobei das Verfahren umfasst:
Verabreichen an das Individuum, das hierfür einen Bedarf hat, einer Menge eines Adeno-assoziierten viralen Vektors, der ausgewählt ist, AAV2 zu sein, und eine humane TMPRSS3-Nukleinsäuresequenz, welche ausgewählt ist aus der Gruppe bestehend aus SEQ ID NO:1 und einer Nukleinsäuresequenz, die mindestens 90% Sequenzidentität mit der Nukleinsäure von SEQ ID NO:1 aufweist, und einen Humanen Cytomegalievirus (HCMV)-Promotor, der operativ mit der Nukleinsäure gekoppelt ist, aufweist.

4. Pharmazeutische Zusammensetzung zur Verwendung nach Anspruch 3, wobei die wirksame Menge wirksam ist, um Genexpression bei Haarzellen im Ohr des Individuums wiederherzustellen.

5. Pharmazeutische Zusammensetzung zur Verwendung nach einem von Anspruch 3 oder Anspruch 4, wobei der Adeno-assoziierte virale Vektor durch ein Injektionsverfahren verabreicht wird, und wobei das Injektionsverfahren ausgewählt ist aus der Gruppe bestehend aus Cochleostomie, Injektion in die Rundfenstermembran, Injektion in den Endolymphsack, Injektion in die Scala media, Canalostomie, Injektion in die Scala media über den Endolymphsack, oder eine beliebige Kombination derselben.

6. Pharmazeutische Zusammensetzung zur Verwendung nach Anspruch 3, wobei das Individuum einen oder mehrere mit Hörverlust verbundene genetische Risikofaktoren aufweist.

7. Pharmazeutische Zusammensetzung zur Verwendung nach Anspruch 6, wobei einer der genetischen Risikofaktoren ausgewählt ist aus der Gruppe bestehend aus einer Mutation im TMPRSS3-Gen oder einer Mutation im LOXHD1-Gen.

8. Pharmazeutische Zusammensetzung zur Verwendung nach Anspruch 6, wobei das Individuum keine klinischen Indikatoren für Hörverlust zeigt.

9. Transgene Maus, die so beschaffen ist, dass sie eine oder mehrere Knock-in-Mutationen aufweist, die Hörverlust verursachen, wobei die Mutationen jeweils einer Mutation in einem humanen TMPRSS3 entsprechen, von der bekannt ist, dass sie Hörverlust verursacht, wobei die eine oder mehreren Knock-in-Mutationen ausgewählt sind aus der Gruppe bestehend aus: IVS4AS, G-A, -6; 8-BP DEL, SATELLITEN-WIEDERHOLUNG INS; 1-BP DEL207C; c.753G>C (p.Trp251Cys); c.308A>G (p.Asp103Gly); c.1211C>T (p.Pro404Leu); c.647G>T (p.Arg216Leu); c.579dupA (p.Cys194Metfs); c.1192C>T (p.Gln398Ter); c.323-6G>A; c.916G>A (p.Ala306Thr); c.208deIC (p.His70Thrfs); c.1276G>A (p.Ala426Thr); c.413C>A (p.Ala138Glu); c.325C>T (p.Arg109Trp); c.346G>A (p.V116M); c.727G>A (p.G243R); und c.1156T>C (p.C386R).

10. Transgene Maus nach Anspruch 9, wobei die Mutation c.916G>A (p.Ala306Thr) ist.

## Revendications

1. Composition pharmaceutique destinée à être utilisée dans un procédé de traitement ou de prévention de la perte auditive chez un sujet en ayant besoin, comprenant :
un vecteur viral adéno-associé choisi pour être AAV2 ayant une séquence d'acide nucléique de TMPRSS3 humain choisi dans le groupe constitué par SEQ ID n° 1 et une séquence d'acide nucléique ayant au moins 90 % d'identité de séquence à l'acide nucléique de SEQ ID n° 1, et un promoteur de cytomégalovirus humain (HCMV) lié opérationnellement à l'acide nucléique.

2. Composition pharmaceutique destinée à être utilisée selon la revendication 1, dans laquelle le titre de vecteur viral est de 1,1 × 10¹³ copies de génome par millilitre (GC/mL), tel que déterminé par pPCR.

3. Composition pharmaceutique destinée à être utilisé selon la revendication 1 ou 2, le procédé comprenant :
l'administration au sujet en ayant besoin d'une quantité d'un vecteur viral adéno-associé choisi pour être AAV2 et ayant une séquence d'acide nucléique de TMPRSS3 humain choisie dans le groupe constitué par SEQ ID n° 1 et une séquence d'acide nucléique ayant au moins 90 % d'identité de séquence à l'acide nucléique de SEQ ID n° 1, et un promoteur de cytomégalovirus humain (HMCV) lié opérationnellement à l'acide nucléique.

4. Composition pharmaceutique destinée à être utilisée selon la revendication 3, dans laquelle la quantité efficace est efficace pour restaurer l'expression génique des cellules pileuses dans l'oreille du sujet.

5. Composition destinée à être utilisée selon l'une quelconque de la revendication 3 ou la revendication 4, dans laquelle le vecteur viral adéno-associé est administré par un procédé d'injection et dans laquelle le procédé d'injection est choisi dans le groupe constitué par la cochléostomie, l'injection dans la membrane de fenêtre ronde, l'injection dans le sac endolymphatique, l'injection dans la média scala, la canalostomie, l'injection dans la média scala via le sac endolymphatique ou une quelconque de leurs combinaisons.

6. Composition pharmaceutique destinée à être utilisée selon la revendication 3, dans laquelle le sujet a un ou plusieurs facteurs de risque génétique associés avec la perte auditive.

7. Composition pharmaceutique destinée à être utilisée selon la revendication 6, dans laquelle un des facteurs de risque génétique est choisi dans le groupe constitué par une mutation dans le gène TMPRSS3 ou une mutation dans le gène LOXHD1.

8. Composition pharmaceutique destinée à être utilisé selon la revendication 6, dans laquelle le sujet ne présente aucun indicateur clinique de perte auditive.

9. Souris transgénique conçue pour avoir une ou plusieurs mutations knock-in qui entraînent la perte auditive, les mutations correspondant chacune à une mutation dans un TMPRSS3 humain connues pour entraîner la perte auditive, dans laquelle les une ou plusieurs mutations knock-in sont choisies dans le groupe constitué par : IVS4AS, G-A, -6 ; 8-BP DEL, SATELLITE REPEAT INS ; 1-BP DEL 207C ; c.753G>C (p. Trp251Cys) ; c.308A>G (Asp103Gly) ; c.1211C>T (p.Pro404Leu) ; c647G>T (p.Arg216Leu) ; c.579dupA (p.Cys194Metfs) ; c1192C>T (p.Gln398Ter) ; c.323-6G>A ; c.916G>A (pAla306Thr) ; c.208delC (p.His70Thrfs) ; c.1276G>A (p.Ala426Thr) ; c.413C>A (p.Ala138Glu) ; c.325C>T (p.Arg109Trp) ; c.346G>A (p.V116M) ; c.727G>A (p.G243R) ; et c.1156T>C (p.C386R).

10. Souris transgénique selon la revendication 9, dans laquelle la mutation est c.916G>A (p.Ala306Thr).
